# EUROPEAN PATENT SPECIFICATION

(11) **EP 2 757 151 B1**
(45) Date of publication and mention of the grant of the patent: **30.08.2017**
(21) Application number: 12831055.4
(22) Date of filing: 22.06.2012
(51) Int. Cl.: C12N 9/99, A01N 1/02, C07C 309/81, C07C 309/88, G01N 1/28

(54) **METHOD FOR INHIBITING PROTEASE IN BIOLOGICAL SAMPLE CONTAINING PANCREATIC JUICE COMPONENT**
VERFAHREN ZUR PROTEASEHEMMUNG IN BIOLOGISCHEN PROBEN MIT EINER PANKREASSAFTKOMPONENTE
PROCÉDÉ D'INHIBITION D'UNE PROTÉASE DANS UN ÉCHANTILLON BIOLOGIQUE CONTENANT UN COMPOSANT DU SUC PANCRÉATIQUE

(30) Priority: 13.09.2011 JP 2011199357
(43) Date of publication of application: 23.07.2014
(73) Proprietor: Olympus Corporation, Tokyo 192-8507 (JP)
(72) Inventor: KATAOKA, Rie, Tokyo 192-8507 (JP); NAKATA, Mari, Tokyo 192-8507 (JP); MORIYA, Nao, Tokyo 192-8507 (JP)
(74) Representative: Schicker, Silvia
(86) International application number: PCT/JP2012/066004
(87) International publication number: WO 2013/038767

(56) References cited:
- EP-A1- 2 696 202
- JP-A- 2005 525 126
- JP-A- 2007 222 062
- US-A1- 2009 215 182
- WANDSCHNEIDER S. ET AL.: 'Autoimmune pancreatic disease: preparation of pancreatic juice for proteome analysis' ELECTROPHORESIS vol. 22, no. 20, 2001, pages 4383 - 4390, XP055147890
- KAINO S. ET AL.: 'Two-dimensional zymography for analysis of proteolytic enzymes in human pure pancreatic juice' ELECTROPHORESIS vol. 19, no. 5, 1998, pages 782 - 787, XP009029643
- PAULO JA. ET AL.: 'Optimized sample preparation of endoscopic collected pancreatic fluid for SDS-PAGE analysis' ELECTROPHORESIS vol. 31, no. 14, 2010, pages 2377 - 2387, XP055092467
- KAZUHITO EHARA: 'Suiekichu ni Okeru Lipase Kassei no Shikkatsu no Yoin ni Tsuite no Kenkyu' THE JAPANESE JOURNAL OF MEDICAL TECHNOLOGY, EISEI KENSA vol. 31, no. 7, 1982, pages 1039 - 1042, XP008173234

## Description

### TECHNICAL FIELD

The present invention relates to a method for inhibiting protease in a biological sample containing pancreatic juice components, and a protease inhibitor mixture, as defined in the claims.

### BACKGROUND ART

Bodily fluids serve as important biological samples for determining the states of organs. Pancreatic juice is also an important biological sample for determining the state of the pancreas, and is used in cytodiagnosis, bicarbonate assay, bacteriological examinations and examinations using markers composed of proteins, nucleic acids and the like. Wide-ranging searches for novel markers for use in pancreatic cancer and other pancreas-related diseases are being made in research fields as well.

Since pancreatic juice is a digestive juice, it contains various digestive enzymes. Although these digestive enzymes are present in an inactive state in the pancreas, they are known to be activated after having been discharged into the duodenum. Digestive enzymes in pancreatic juice undergo a degradatory cascade reaction induced by intestinal enterokinase secreted from duodenal epithelial cells. Trypsinogen contained in pancreatic juice is activated by intestinal enterokinase resulting in conversion to trypsin, and this trypsin is known to trigger activation of various digestive enzymes (proteases) such as chymotrypsinogen and proesterase. Furthermore, trypsinogen is said to account for roughly 12% of the total protein content of pancreatic juice (see, for example, Non-Patent Document 2). As a result of activation of these various proteases, biomolecules such as proteins, nucleic acids, lipids or cells contained in pancreatic juice are degraded and denatured following discharge into the duodenum. Consequently, in the case of utilizing pancreatic juice discharged into the duodenum in testing and research such as cytodiagnosis or biomolecular assays, there is concern over measurements being unable to be performed accurately as a result of the target cells or proteins and the like present in pancreatic juice being affected by proteases. Consequently, it is important to inhibit the activity of proteases as much as possible in pancreatic juice discharged into the duodenum.

One example of a conventional method for reducing the activity of various degradatory enzymes present in pancreatic juice and duodenal juice consists of suppressing their activity by maintaining collected pancreatic juice in a chilled state so as to be outside the optimum temperature range of enzyme activity. In general, collected pancreatic juice and the like is required to immediately be placed in ice, and testing on that pancreatic juice and the like is required to be carried out promptly (see, for example, Non-Patent Document 1). In this method, however, there is the potential for enzyme reactions to resume if the temperature of the pancreatic juice and the like rises. Consequently, it is necessary to constantly maintain the collected pancreatic juice at a low temperature, thereby making handling bothersome. In addition, since degradation proceeds even at low temperatures depending on the type of protein, simply maintaining at a low temperature is inadequate for suppressing degradation and denaturation of pancreatic juice-derived components.

In order to measure the protein in pancreatic juice, a method has been disclosed by which 0.2 mL of aprotinin is added to 0.5 mL of pancreatic juice collected with an endoscope followed by placing in frozen storage (see, for example, Non-Patent Document 2). Nearly all protease activity in the pancreatic juice can be suppressed during the storage period by placing in frozen storage, thereby enabling cells or proteins and the like present in the pancreatic juice to be preserved in a stable state. However, since frozen pancreatic juice is required to be thawed prior to measurement, there are cases in which degradation of the cells or proteins resumes after thawing. Moreover, there is also the risk of the molecules targeted for analysis per se being damaged during frozen storage.

In addition, a method for preserving biological samples, and particularly blood, has been disclosed in which blood is collected directly into a container containing at least two types of protease inhibitors (see, for example, Patent Document 1). As a result of immediately contacting the collected blood with the protease inhibitors, degradation and the like of proteins in the blood can be prevented. In this method, a serine protease inhibitor and other protease inhibitors are used, and blood is collected into a container containing, for example, a serine protease inhibitor such as AEBSF, aprotinin or leupeptin, and a cysteine protease inhibitor such as E-64. Wandschneider et al. Electrophoresis, 2001, 22 (20) : 4383-4390; and Kaino et al. Electrophoresis, 1998, 19 (5) : 782-787 disclose that proteolysis caused by protease in a biological sample can be inhibited by adding PMSF and/or AEBSF.
JP 2007-222062 describes a mixture of protease inhibitors containing PMSF and TLCK.
JP 2005-525126 discloses a kit for storing a biological sample,
wherein a protease inhibitor is contained in a storage container provided with a reserving section for reserving a biological sample.

### Prior Art Documents

### Patent Documents

Patent Document 1: Japanese Patent No. 4496407

### Non-Patent Documents

Non-Patent Document 1: Katsunuma, et al., "Analysis", 1978, Vol. 10, pp. 682-689
Non-Patent Document 2: Yokoyama, et al., Pancreas, 2002, Vol. 24, No. 4, pp. 344-347

### DISCLOSURE OF THE INVENTION

### Problems to be Solved by the Invention

A method consisting of the addition of protease inhibitor to a collected body fluid is thought to be useful for stably and easily handing collected pancreatic juice and duodenal juice in the same manner as the method described in Patent Document 1.

However, the types of proteases contained differ considerably between blood and pancreatic juice. Consequently, even if a protease inhibitor effective for lowering the activity of proteases present in blood as described in Patent Document 1 is added to a body fluid containing pancreatic juice components such as duodenal juice, this does not mean that protease activity in the body fluid can be suppressed.

An object of the present invention is to provide a protease inhibitor mixture capable of effectively suppressing the activity of protease present in a biological sample containing pancreatic juice components such as pancreatic juice or duodenal juice, and a method for inhibiting protease present in a biological sample containing pancreatic juice components that uses this protease inhibitor mixture.

### Means for Solving the Problems

As a result of conducting extensive studies to solve the aforementioned problems, the inventors of the present invention found that a protease inhibitor mixture comprising PMSF, AEBSF, and TLCK demonstrates the highest activity inhibitory effects against proteases contained in pancreatic juice, thereby leading to completion of the present invention.
(1) A first aspect of the present invention is a method for inhibiting protease in a biological sample containing pancreatic juice components, comprising: adding protease inhibitors comprising PMSF, AEBSF, and TLCK to the biological sample containing pancreatic juice components, thereby inhibiting protease enzyme activity in the biological sample.
(2) The method for inhibiting protease in a biological sample containing pancreatic juice components of (1) above, wherein the protease inhibitor may further comprise one or more types of compounds selected from the group consisting of p-APMSF, 4-(fluorosulfonyl)benzoic acid, 3-(fluorosulfonyl)benzoic acid, 2-aminobenzenesulfonyl fluoride, 3-aminobenzenesulfonyl fluoride, 4-aminobenzenesulfonyl fluoride, 2-nitrobenzenesulfonyl fluoride, 3-nitrobenzenesulfonyl fluoride and 4-nitrobenzenesulfonyl fluoride.
(3) The method for inhibiting protease in a biological sample containing pancreatic juice components of (1) above, wherein the protease inhibitors comprise PMSF, and AEBSF and further optionally p-APMSF.
(4) The method for inhibiting protease in a biological sample containing pancreatic juice components of any of (1) to (3) above, wherein PMSF, AEBSF or p-APMSF is preferably added to the biological sample so that the final concentration of PMSF is 1 mM or higher, the final concentration of AEBSF is 4 mM or higher, the final concentration of TLCK is 0.1 mM or higher, and optionally the final concentration of p-APMSF is 2 mM or higher.
(6) The method for inhibiting protease in a biological sample containing pancreatic juice components of (1) above, wherein the protease inhibitors may further comprise TPCK.
(7) The method for inhibiting protease in a biological sample containing pancreatic juice components of any of (1) to (6) above, wherein the biological sample is preferably pancreatic juice or duodenal juice.
(8) A second aspect of the present invention is a protease inhibitor mixture comprising PMSF, AEBSF, and TLCK.
(9) The protease inhibitor mixture of (8) above may optionally further comprise a compound selected from the group consisting of p-APMSF, 4-(fluorosulfonyl)benzoic acid, 3-(fluorosulfonyl)benzoic acid, 2-aminobenzenesulfonyl fluoride, 3-aminobenzenesulfonyl fluoride, 4-aminobenzenesulfonyl fluoride, 2-nitrobenzenesulfonyl fluoride, 3-nitrobenzenesulfonyl fluoride and 4-nitrobenzenesulfonyl fluoride.
(10) The protease inhibitor mixture of (8) above may preferably further comprise p-APMSF.

### Effects of the Invention

The protease inhibitory mixture according to the second aspect of the present invention is able to extremely effectively suppress protease contained in a biological sample containing pancreatic juice components such as pancreatic juice or duodenal juice. Consequently, the method for inhibiting protease in a biological sample containing pancreatic juice components according to the first aspect of the present invention that uses this protease inhibitor mixture is able to effectively suppress protease activity in a biological sample containing pancreatic juice components. Accordingly, according to this inhibition method, degradation of pancreatic juice-derived components is suppressed, and biological samples containing pancreatic juice components can be stably preserved.

### BRIEF DESCRIPTION OF THE DRAWINGS

FIG. 1 is a drawing showing the chemical formulas of PMSF, AEBSF and p-APMSF.
FIG. 2 is a graph indicating fluorescence measured from various pancreatin solutions in Example 1.
FIG. 3 is a graph indicating the results of measuring S100P concentrations in various sample solutions in Example 2.
FIG. 4 is a graph indicating fluorescence measured from various sample solutions in Example 3.
FIG. 5 is a graph indicating fluorescence measured from various sample solutions in Example 4.
FIG. 6 is a graph indicating fluorescence measured from various sample solutions in Example 5.
FIG. 7 is a graph indicating fluorescence measured from various sample solutions in Example 5.
FIG. 8 is a graph indicating the results for protease activity (relative value: %) of various sample solutions in Example 6.
FIG. 9 is a graph indicating the results for protease activity (relative value: %) of various sample solutions to which PMSF was added in Example 7.
FIG. 10 is a graph indicating the results for protease activity (relative value: %) of various sample solutions to which AEBSF was added in Example 7.
FIG. 11 is a graph indicating the results for protease activity (relative value: %) of various sample solutions to which p-APMSF was added in Example 7.
FIG. 12 is a graph indicating fluorescence measured from various sample solutions to which one type of protease inhibitor was added in Example 8.
FIG. 13 is a graph indicating fluorescence measured from various sample solutions to which two types of protease inhibitors were added in Example 8.
FIG. 14 is a graph indicating fluorescence measured from various sample solutions to which various types of protease inhibitors were added in Example 9.

### BEST MODE FOR CARRYING OUT THE INVENTION

Pancreatic juice refers to a body fluid that is discharged from the pancreatic duct. In the present invention and description of the present application, a biological sample containing pancreatic juice components refers to a sample containing body fluid that contains components derived from pancreatic juice. Examples of body fluid that contains components derived from pancreatic juice include pancreatic juice collected directly from the pancreas with a catheter and liquid collected in the duodenum (duodenal juice). Duodenal juice contains pancreatic juice as well as bile similarly discharged from the papillary region, liquid inherently present in the duodenum, and blood. Pancreatic juice and duodenal juice can be collected by ordinary methods.

### <Protease Inhibitor for Biological Sample Containing Pancreatic Juice Components>

The protease inhibitor for a biological sample containing pancreatic juice components according to the the present disclosure(to also be referred to as the "protease inhibitor of the present disclosure") is characterized as a compound that has a sulfonyl fluoride group, has protease inhibitory activity, and is added to a biological sample containing pancreatic juice components in order to inhibit protease present in the biological sample.

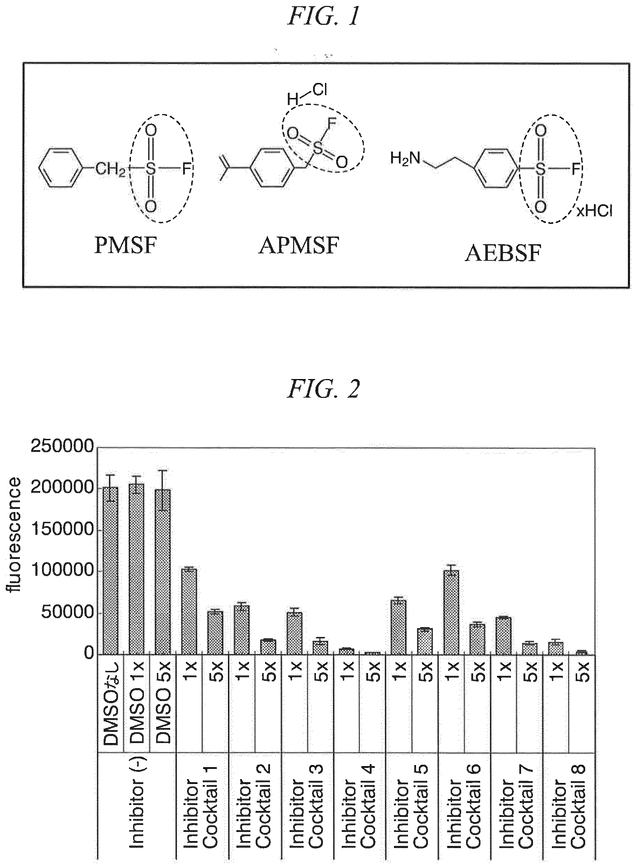

Although there are no particular limitations on the protease inhibitor of the present disclosure provided it is a compound that has a sulfonyl fluoride group and has protease inhibitory activity, a compound having a structure in which a sulfonyl fluoride group is bound directly to a benzene ring or through a hydrocarbon group having 1 to 6 carbon atoms is preferable, is preferably phenylmethylsulfonyl fluoride (PMSF), 4-(2-aminoethyl)-benzenesulfonyl fluoride (AEBSF), p-amidinophenylmethane sulfonyl fluoride hydrochloride (p-APMSF), 4-(fluorosulfonyl)benzoic acid (CAS No. 455-26-5), 3-(fluorosulfonyl)benzoic acid (CAS No. 454-95-5), 2-aminobenzenesulfonyl fluoride (CAS No. 392-86-9), 3-aminobenzenesulfonyl fluoride (CAS No. 368-50-3), 4-aminobenzenesulfonyl fluoride (CAS No. 98-62-4), 2-nitrobenzenesulfonyl fluoride (CAS No. 433-98-7), 3-nitrobenzenesulfonyl fluoride (CAS No. 349-78-0) or 4-nitrobenzenesulfonyl fluoride (CAS No. 349-96-2), and more preferably PMSF, AEBSF or p-APMSF. FIG. 1 shows the chemical formulas of PMSF, AEBSF and p-APMSF. In FIG. 1, the functional groups encircled by dotted lines are sulfonyl fluoride groups.

The protease inhibitor of the present disclosure may be an active ingredient of a mixture composed of a plurality of types of protease inhibitors. The protease inhibitor mixture may be composed only of the protease inhibitor of the present invention as defined in the claims or may be a mixture of the protease inhibitor of the present invention and other protease inhibitors. There are no particular limitations on the other protease inhibitors provided they do not impair the protease inhibitory activity of the protease inhibitor of the present invention as defined in the claims, and examples thereof include peptide-based protease inhibitors such as aprotinin, leupeptin, antipain, chymostatin, elastatinal and antithrombin; chelating agents such as EDTA; elastase inhibitor, TPCK, trypsin inhibitor, ecotin and E. coli. In addition, pancreatitis therapeutic drugs such as gabexate mesilate (Foy), camostat mesilate (Foipan), nafamostat mesilate (Futhan) or ulinastatin can also be used.

Other protease inhibitors used in combination with the protease inhibitor of the present invention as defined in the claims preferably consist of at least one type of protease inhibitor having a sulfonyl group, and more preferably consist of amino acid chloromethyl ketones. There are no particular limitations on the amino acid chloromethyl ketone provided it has protease inhibitory activity. Examples of amino acid chloromethyl ketones include N-a-tosyl-L-lysine chloromethyl ketone (TLCK) and N-a-tosyl-L-phenylalanine chloromethyl ketone (TPCK). In the present invention, the protease inhibitors comprise TLCK, and optionally in addition and TPCK.

A mixture of the protease inhibitors of the present invention as defined in the claims, preferably contains two or more types of the protease inhibitor of the presentdisclosure, but in any case PMSF, AEBSF and TLCK.

### <Method for Inhibiting Protease in Biological Sample Containing Pancreatic Juice Components>

The method of inhibiting protease in a biological sample containing pancreatic juice components according to the first aspect of the present invention (to also be referred to as the "protease inhibition method of the present invention") is characterized by adding the protease inhibitors as defined in the claims to the biological sample, thereby inhibiting protease enzyme activity in a biological sample containing pancreatic juice components. The protease inhibitor mixture of the present invention as defined in the claims has extremely high inhibitory activity against protease contained in pancreatic juice and duodenal juice in comparison with other protease inhibitors. Consequently, by adding the protease inhibitors of the present invention as defined in the claims to a biological sample containing pancreatic juice components, degradation of pancreatic juice components by protease derived from pancreatic juice and duodenal juice present in the biological sample can be easily and effectively suppressed.

The biological sample containing pancreatic juice components used in the protease inhibition method of the present invention is a sample that contains body fluid containing components derived from pancreatic juice. For example, it may be composed only of body fluid containing components derived from pancreatic juice, a liquid obtained by diluting the body fluid with a suitable buffer and the like, or a liquid obtained by adding various types of additives to the body fluid or a diluted liquid thereof. Examples of additives include surfactants, nuclease inhibitors, pH adjusters and pH indicators. The biological sample containing pancreatic juice components used in the protease inhibition method of the present invention is preferably a sample that contains pancreatic juice or duodenal juice. Specific examples thereof include pancreatic juice, duodenal juice, diluted pancreatic juice or duodenal juice, and liquids obtained by adding the various types of additives listed above thereto.

In the protease inhibition method of the present invention, although the protease inhibitor of the present invention may be added to a biological sample containing pancreatic juice components that has been stored after having been collected from the body, the time interval from the time the body fluid containing pancreatic juice components is collected from the body until the time the protease inhibitors of the present invention as defined in the claims are added thereto is preferably short, and the protease inhibitors of the present invention as defined in the claims are particularly preferably added to the pancreatic juice or duodenal juice immediately after having been collected from the body.

A wide variety of proteases are contained in pancreatic juice, examples of which include serine protease, metalloprotease and carboxypeptidase. Consequently, in the case of protease inhibitors conventionally used for pancreatic juice and the like, protease inhibitory effects obtained in the case of adding only one type of protease inhibitor are extremely limited, and even in the case of combining several types of protease inhibitors, the obtaining of adequate protease inhibitory effects has yet to be reported. In contrast, the protease inhibitors of the present invention as defined in the claims allow obtaining adequate protease inhibitory effects by suitably adjusting the amount added.
The combined use of two or more types is preferable. In addition, one type or two or more types of different protease inhibitors may be used together with the protease inhibitors of the present invention as defined in the claims. The protease inhibitors of the present invention used in the protease inhibition method of the present invention comprise PMSF, AEBSF and TLCK, optionally in combination with p-APMSF. Since pancreatic juice contains various proteases having different activities, protease activity in a biological sample can be expected to be more reliably suppressed by using a mixture of a plurality of protease inhibitors. Moreover, protease activity can be expected to be suppressed by combining at a lower concentration than in the case of using alone.

There are no particular limitations on the amount of protease inhibitor added to a biological sample containing pancreatic juice components provided it is an amount at which protease inhibitory effects are demonstrated by the protease inhibitor, and can be suitably adjusted in consideration of such factors as the type of biological sample containing pancreatic juice components or the type of protease inhibitor. For example, in the case of adding only PMSF as protease inhibitor to a biological sample containing pancreatic juice components, the PMSF is added so that the final concentration thereof is 1 mM or higher, preferably 5 mM or higher and more preferably 10 mM or higher. In addition, in the case of using only AEBSF as protease inhibitor, AEBSF can be added to a biological sample containing pancreatic juice components so that the final concentration thereof is 4 mM or higher, preferably 10 mM or higher and more preferably 20 mM or higher. In the case of using only p-APMSF as protease inhibitor, it can be added to a biological sample containing pancreatic juice components so that the final concentration thereof is 2 mM or higher, preferably 5 mM or higher and more preferably 10 mM or higher.

In addition, in the case of adding TLCK together with the protease inhibitors as defined in the claims (the protease inhibitors of the present invention), TLCK can be added so that the final concentration thereof is 0.1 mM or higher, preferably 1 mM or higher, more preferably 5 mM or higher and even more preferably 10 mM or higher. In addition, in the case of further adding only TPCK as the other protease inhibitor, TPCK can be added so that the final concentration thereof is 0.1 mM or higher, preferably 1 mM or higher, more preferably 5 mM or higher, and even more preferably 10 mM or higher.

In the protease inhibition method of the present invention, the protease inhibitors of the present invention as defined in the claims added to a biological sample containing pancreatic juice components may be in the form of a solid such as a powder or granules, or may be a protease inhibitor solution obtained by dissolving in a suitable buffer and the like. In addition, the protease inhibitors of the present invention as defined in the claims may be added simultaneously to a biological sample containing pancreatic juice components, or one may be first added followed by adding the other. From the viewpoint of being able to adequately demonstrate inhibitory effects against protease present in a biological sample, the protease inhibitors are preferably added simultaneously.

Since the activity of proteases contained in a biological sample containing pancreatic juice components is effectively inhibited by adding the protease inhibitors of the present invention as defined in the claims to the biological sample, degradation, denaturation and the like of pancreatic juice components contained in the biological sample are remarkably suppressed. Consequently, a biological sample containing pancreatic juice components in which protease has been inhibited by the protease inhibition method of the present invention allows biological components derived from pancreatic juice to be preserved more stably. In addition, analysis accuracy can be improved and highly reliable results can be obtained by analyzing pancreatic juice components obtained from biological samples containing pancreatic juice components in which protease has been inhibited by the protease inhibition method of the present invention as defined in the claims.

A biological sample containing pancreatic juice components in which protease has been inhibited by the protease inhibition method of the present invention can be used as a measurement sample for various types of testing in the same manner as other biological samples. There are no particular limitations on the tested substance provided it is a biological component expected to be contained in pancreatic juice or duodenal juice, and it may be a protein, nucleic acid such as DNA and RNA, or cell. For example, the biological sample can be used in various types of protein analyses such as ELISA, immunochromatography, two-dimensional electrophoresis, western blotting or mass spectrometry, various types of nucleic acid analyses such as PCR, RT-PCR and probe-based hybridization, and various types of cytoanalyses in the manner of cell counting or cytodiagnosis.

### <Kit for Preserving Biological Sample Containing Pancreatic Juice Components>

The kit for preserving a biological sample containing pancreatic juice components of the present disclosure(also referred to as the "preservation kit of the present disclosure") contains at least one type of protease inhibitor having a sulfonyl fluoride group (namely, the protease inhibitors of the present invention as defined in the claims), and is used to preserve biological samples containing pancreatic juice components. The use of this kit makes it possible to more easily inhibit protease present in a biological sample containing pancreatic juice components.

In addition, the protease inhibitors of the present invention contained in the preservation kit of the present disclosure may be in the form of a freeze-dried solid, may be in the form of a tablet or granules obtained by molding a freeze-dried powder with a suitable vehicle, or may be in the form of a protease inhibitor solution obtained by dissolving in a suitable buffer.

The preservation kit of the present disclosure may further contain a buffer for diluting a collected body fluid, other protease inhibitors, surfactant, pH adjuster or pH indicator and the like. Various types of additives such as a surfactant, pH adjuster or pH indicator may be preliminarily dissolved in a dilution buffer.

In addition, the preservation kit of the present disclosure may also contain a cap capable of joining to an opening of a container filled with a prepared sample containing pancreatic juice components, that allows a fixed amount of the sample (obtained by adding various types of protease inhibitors contained in the preservation kit of the present disclosure as well as other components as necessary to a biological sample containing pancreatic juice components) to be dropped from the container.

The preservation kit of the present disclosure may further contain a storage container provided with a storage portion for storing body fluid such as pancreatic juice or duodenal juice collected from the body. In this case, the protease inhibitors of the present invention and other protease inhibitors are preferably preliminarily contained in the storage portion. In the case graduations are formed in the storage container, the amount of biological sample added to the storage container (total amount of biological sample and protease inhibitor in the case protease inhibitor has been preliminarily filled into the storage container) can be confirmed visually, thereby enabling the final concentration of protease inhibitor to be roughly determined at a glance.

In addition, biological samples containing pancreatic juice components are typically collected transendoscopically. Therefore, a collection tool for collecting a biological sample containing pancreatic juice components transendoscopically may also be contained as a component of the preservation kit of the presentdisclosure. Examples of this collection tool include the combination of a syringe and catheter capable of being inserted into an endoscope apparatus, and a probe provided with an absorber on the end thereof capable of being inserted into an endoscope apparatus. An example of a catheter capable of being inserted into an endoscope apparatus is the specimen collection cube described in Japanese Unexamined Patent Application, First Publication No. 2011-5009. These collection tools preferably have the aforementioned storage portion preliminarily filled with protease inhibitor. Examples

### [Example 1] (Not claimed)

A search was made of protease inhibitor cocktails that demonstrate highly effective inhibitory effects against proteases present in pancreatic juice from among eight types of commercially available protease inhibitor cocktails in order to determine the protease inhibitor having the greatest effect against proteases present in pancreatic juice. The protease inhibitor cocktails used consisted of Complete (Roche, Inhibitor Cocktail 1), Halt Protease Inhibitor Cocktail (Thermo, Inhibitor Cocktail 2), Protease Inhibitor Cocktail (Sigma, Inhibitor Cocktail 3), Protease Inhibitor Mix (GE, Inhibitor Cocktail 4), Protease Inhibitor Cocktail Set I (Merck, Inhibitor Cocktail 5), Protease Inhibitor Cocktail Set II (Merck, Inhibitor Cocktail 6), Protease Inhibitor Cocktail Set III (Merck, Inhibitor Cocktail 7), and Protease Inhibitor Cocktail (Biovision, Inhibitor Cocktail 8).

After adding each protease inhibitor cocktail so that the final concentration thereof was equal to manufacturer's recommended concentration (1×) or a concentration equal to 5 times the recommended concentration (5×), protease activity was measured using a simulated artificial pancreatic juice in the form of "pancreatin", a digestive enzyme produced from porcine pancreas in which pancreatic enzymes are present in an activated state. Protease activity was measured using EnzCheck Protease Assay Kits (Molecular Probes). More specifically, fluorescent-labeled casein provided with the kits was added to a pancreatin solution to which each protease inhibitor cocktail had been added, and after incubating for 2 hours at 37°C, fluorescence was measured at a fluorescence wavelength of Ex/Em = 485/535 nm. In addition, a sample solution in which fluorescent-labeled casein was added directly to pancreatin (inhibitor (-), DMSO (-)), a sample solution in which DMSO was added to pancreatin so that the added amount of DMSO was the same as in the case of adding each protease inhibitor cocktail at the manufacturer's recommended concentration (1×) (inhibitor (-), DMSO 1×), and a sample solution in which DMSO was added to pancreatin so that the added amount of DMSO was the same as in the case of adding each protease inhibitor cocktail at a concentration equal to five times the manufacturer's recommended concentration (5x) (inhibitor (-), DMSO 5×) were prepared for use as controls, and these sample solutions were measured for protease activity in the same manner as the protease inhibitor cocktails.

The results of measuring fluorescence of each pancreatin solution are shown in FIG. 2. Fluorescence plotted on the vertical axis represents the degraded amount of casein, and that value was estimated to be the value of protease activity. According to these results, Inhibitor Cocktail 4 (GE, Protease Inhibitor Mix) was determined to demonstrate the greatest inhibitory effect.

### [Example 2] (Not claimed)

A study was conducted of the manner in which the storage stability of protein in pancreatic juice is affected by inhibition of protease activity. The degree to which S100P (a calcium-bound protein typically contained in pancreatic juice) is preserved in the case of adding or not adding protease inhibitor to a solution consisting of a mixture of S100P and pancreatin (simulated pancreatic juice) was investigated.

More specifically, a 25 ng/mL S100P (standard) solution (Sample Solution 1), a solution containing 25 ng/mL S100P (standard) and 1 mg/mL of pancreatin (Sample Solution 2), a solution containing 25 ng/mL of S100P (standard) and 1 mg/mL of pancreatin, and further containing Protease Inhibitor Mix (GE) at twice the recommended concentration (2x) (Sample Solution 3), and a solution containing 25 ng/mL of S100P (standard) and 1 mg/mL of pancreatin, and further containing Complete (Roche) at five times the recommended concentration (5x) (Sample Solution 4) were first prepared. Each sample solution was prepared using the buffer provided with the Circulex S100P ELISA Kit (Cyclex, Catalog No.: CY-8060). These sample solutions were allowed to react by incubating for 16 hours at 25°C. Subsequently, each sample solution was diluted 10-fold using the buffer provided with the Circulex S100P ELISA Kit (Cyclex, Catalog No.: CY-8060), and S100P was detected in the diluted sample solutions using the Circulex S100P ELISA Kit.

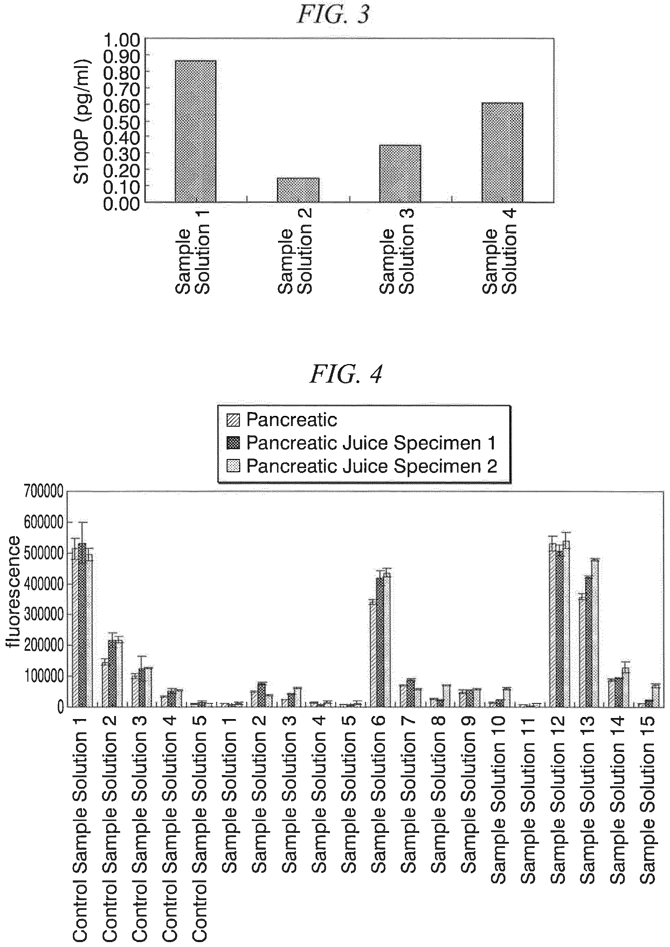

The results of measuring S100P in each sample solution are shown in FIG. 3. As a result, the residual amount of S100P was greater in Sample Solutions 3 and 4 to which protease inhibitor cocktail had been added than in Sample Solution 2 to which protease inhibitor cocktail had not been added. In addition, the residual amount of S100P was greater in Sample Solution 4, to which was added protease inhibitor cocktail having higher protease inhibitory activity than the protease inhibitor cocktail added to Sample Solution 3 (see Example 1), than in Sample Solution 3. On the basis of these results, the addition of protease inhibitor to pancreatic juice was clearly determined to suppress protein degradation in pancreatic juice and allow pancreatic juice to be preserved more stably. In addition, there was suggested to be a correlation between protease activity and detected concentration of S100P.

### [Example 3] (Not claimed)

A study was conducted to determine which protease inhibitor demonstrates the highest level of inhibitory activity against protease present in pancreatic juice among various types of protease inhibitors contained in the inhibitor cocktail demonstrating the highest level of protease inhibitory activity in Example 1.

There are four types of serine protease inhibitors contained in Protease Inhibitor Mix (GE) consisting of aprotinin, leupeptin, PMSF and AEBSF (concentrations are not disclosed). Therefore, protease activity was measured in the same manner as Example 1 using pancreatin and two types of pancreatic juice specimens for 15 combinations of the four types of inhibitors prepared so that the final concentration was equal to the maximum recommended concentration for each protease inhibitor alone. The protease inhibitors added to each sample solution are shown in Table 1. Furthermore, the maximum recommended concentrations consisted of 0.3 µM for aprotinin (Roche), 50 µM for leupeptin (Roche), 1 mM for PMSF (Roche) and 4 mM for AEBSF (Roche). In addition, protease activity was also measured in the same manner for a sample solution to which protease inhibitor was not added (Control Sample Solution 1), a sample solution containing the recommended concentration of Inhibitor Cocktail 1 (Complete, Roche) (Control Sample Solution 2), a sample solution containing Inhibitor Cocktail 1 at 5 times the recommended concentration (5×) (Control Sample Solution 3), a sample solution containing the recommended concentration of Inhibitor Cocktail 4 (Protease Inhibitor Mix, GE) (Control Sample Solution 4), and a sample solution containing Inhibitor Cocktail 4 at five times the recommended concentration (5×) (Control Sample Solution 5).

**[Table 1]**

| | Aprotinin | Leupeptin | PMSF | AEBSF |
|---|---|---|---|---|
| Sample Solution 1 | ○ | ○ | ○ | ○ |
| Sample Solution 2 | ○ | ○ | ○ | |
| Sample Solution 3 | ○ | ○ | | ○ |
| Sample Solution 4 | ○ | | ○ | ○ |
| Sample Solution 5 | | ○ | ○ | ○ |
| Sample Solution 6 | ○ | ○ | | |
| Sample Solution 7 | ○ | | ○ | |
| Sample Solution 8 | ○ | | | ○ |
| Sample Solution 9 | | ○ | ○ | |
| Sample Solution 10 | | ○ | | ○ |
| Sample Solution 11 | | | ○ | ○ |
| Sample Solution 12 | ○ | | | |
| Sample Solution 13 | | ○ | | |
| Sample Solution 14 | | | ○ | |
| Sample Solution 15 | | | | ○ |

The results of measuring fluorescence of each sample solution are shown in FIG. 4. Fluorescence plotted on the vertical axis represents the degraded amount of casein, and that value was estimated to be the value of protease activity. Sample Solution 1 simulates Inhibitory Cocktail 4 and contains all four types of serine protease inhibitors, and hardly any degradation of casein was observed. Sample Solutions 4, 5 and 11 demonstrated protease inhibitory effects nearly equal to that of Sample Solution 1. The protease inhibitors added in common to these three types of sample solutions were PMSF and AEBSF. On the basis of these results, the combination of PMSF and AEBSF was clearly determined to demonstrate a high level of protease inhibitory effects in pancreatic juice. On the other hand, aprotinin and leupeptin, which are peptide-based protease inhibitors used in blood collection tubes as protease inhibitors for use in blood testing, had hardly any effect on protease activity.

### [Example 4] (Not claimed)

PMSF and AEBSF are both compounds that have a sulfonyl fluoride group. Therefore, a study was conducted on the inhibitory effects of protease inhibitors having a sulfonyl fluoride group and protease inhibitors not having a sulfonyl fluoride group. PMSF (Roche), AEBSF (Roche), p-APMSF (Sigma), TLCK (Sigma) and TPCK (Sigma) were used as protease inhibitors. More specifically, a sample solution obtained by adding 1 mM PMSF and 4 mM AEBSF, a sample solution obtained by adding 1 mM PMSF, a sample solution obtained by adding 4 mM AEBSF, a sample solution obtained by adding 5 mM p-APMSF, a sample solution obtained by adding 100 µM TLCK and a sample solution obtained by adding 100 µM TPCK were prepared for use as protease inhibitors, and the protease activity of each sample solution was measured in the same manner as Example 1 using pancreatin and two types of pancreatic juice specimens. In addition, the protease activities of a sample solution to which protease inhibitor was not added (inhibitor (-)), a sample solution containing Inhibitor Cocktail 1 at the recommended concentration (Complete, Roche), a sample solution containing Inhibitor Cocktail 1 at 5 times the recommended concentration (5×), a sample solution containing Inhibitor Cocktail 4 at the recommended concentration (Protease Inhibitor Mix, GE), and a sample solution containing Inhibitor Cocktail 4 at five times the recommended concentration (5×) were also measured in the same manner.

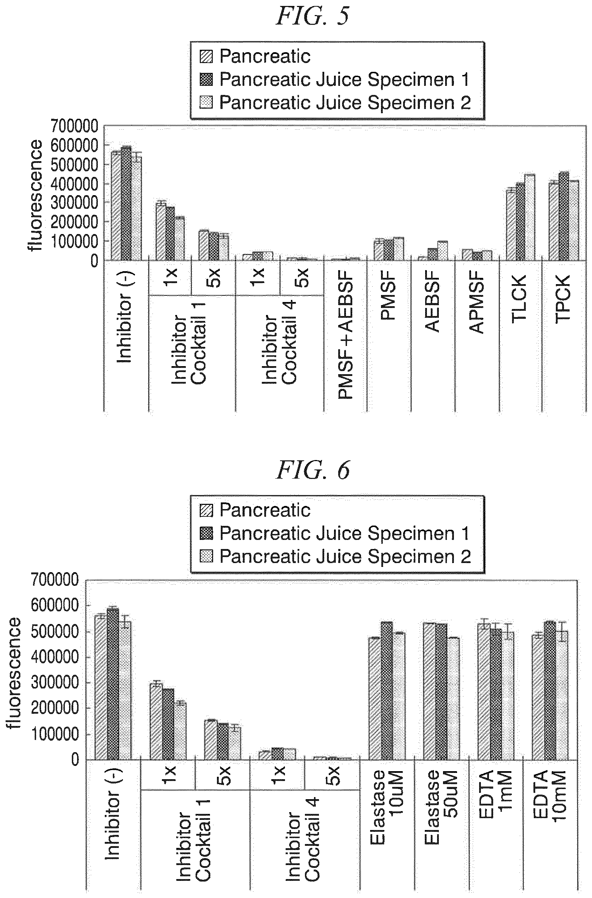

The results of measuring the fluorescence of each sample solution are shown in FIG. 5. Fluorescence plotted on the vertical axis represents the degraded amount of casein, and that value was estimated to be the value of protease activity. The protease inhibitors added to each sample solution are plotted on the horizontal axis. As a result, PMSF, AEBSF and p-APMSF demonstrated high levels of protease inhibitory effects, while TLCK and TPCK were clearly determined to demonstrate low levels of protease inhibitory effects against protease present in pancreatic juice.

As shown in FIG. 1, PMSF, AEBSF and p-APMSF all have chemical structures that are extremely similar. In particular, these compounds all have in common a sulfonyl fluoride group. Consequently, the possibility was suggested that the presence of a sulfonyl fluoride group has a significant effect on inhibition of protease in pancreatic juice. It is thought that a structure having a sulfonyl fluoride group also acts effectively on inhibition of serine proteases, which are known to trigger a degradatory cascade in pancreatic juice.

### [Example 5] (Not claimed)

A study was conducted of the protease inhibitory effects of protease inhibitors not having a sulfonyl fluoride group. Elastase Inhibitor I (Merck), EDTA (Wako Pure Chemical Industries), Aprotinin (Roche), Leupeptin (Roche), TLCK (Sigma) and TPCK (Sigma) were used as protease inhibitors.

More specifically, a sample solution obtained by adding 10 µm to 50 µm Elastase Inhibitor I, a sample solution obtained by adding 1 mM to 10 mM EDTA, a sample solution obtained by adding 0.0003 mM to 0.3 mM Aprotinin, a sample solution obtained by adding 0.005 mM to 5 mM Leupeptin, a sample solution obtained by adding 0.01 mM to 1 mM TLCK or a sample solution obtained by adding 0.01 mM to 1 mM TPCK was prepared for use as protease inhibitor, and the protease activity of each sample solution was measured in the same manner as Example 1 using pancreatin and two types of pancreatic juice specimens. In addition, the protease activities of a sample solution to which protease inhibitor was not added (Inhibitor (-)), a sample solution containing Inhibitor Cocktail 1 at the recommended concentration (Complete, Roche), a sample solution containing Inhibitor Cocktail 1 at five times the recommended concentration (5×), a sample solution containing Inhibitor Cocktail 4 at the recommended concentration (Protease Inhibitor Mix, GE), and a sample solution containing Inhibitor Cocktail 4 at five times the recommended concentration (5x) were also measured in the same manner.

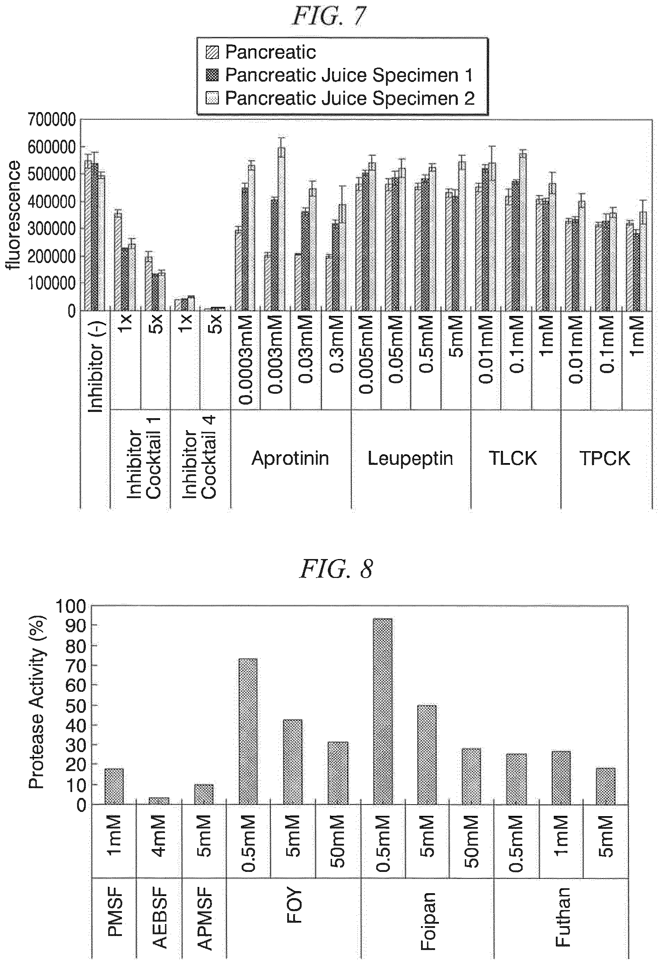

The results of measuring the fluorescence of each sample solution are shown in FIGS. 6 and 7. Fluorescence plotted on the vertical axis represents the degraded amount of casein, and that value was estimated to be the value of protease activity. The protease inhibitors added to each sample solution are plotted on the horizontal axis. As a result, Elastase Inhibitor I, EDTA, Leupeptin and TLCK were observed to demonstrate hardly any inhibitory effects against protease present in pancreatic juice. On the other hand, although Aprotinin was observed to demonstrate weak protease inhibitory effects against pancreatin (simulated pancreatic juice), it was observed to demonstrate hardly any effects against the pancreatic juice specimens. In addition, although TPCK was observed to demonstrate slight protease inhibitory effects, since those inhibitory effects did not increase even if concentration was increased, inhibitory effects against protease present in pancreatic juice were judged to be low.

### [Example 6] (Not claimed)

A study was conducted on the protease inhibitory effects of pancreatitis therapeutic drugs consisting of gabexate mesilate (Foy, Wako Pure Chemical Industries), camostat mesilate (Foipan, Wako Pure Chemical Industries) and nafamostat mesilate (Futhan, BD).

More specifically, sample solutions obtained by respectively adding Foy and Foipan at a final concentration of 0.5 mM to 50 mM and Futhan at a final concentration of 0.5 mM to 5 mM were prepared, and the protease activity of each sample solution was measured in the same manner as Example 1 using pancreatin and two types of pancreatic juice specimens. In addition, the protease activities of a sample solution obtained by adding 1 mM PMSF, a sample solution obtained by adding 4 mM AEBSF and a sample solution obtained by adding 5 mM p-APMSF were also measured in the same manner as protease inhibitors.

The results for the protease activity (relative value: %) of each sample solution are shown in FIG. 8. Protease activity based on a value of 100% for the fluorescence intensity of a sample solution to which protease inhibitor was not added is plotted on the vertical axis, and indicates relative values (%) of protease activity estimated from the fluorescence intensity of each sample solution. The protease inhibitors and final concentrations thereof added to each sample solution are plotted on the horizontal axis. On the basis of these results, the protease inhibitory effects of these pancreatitis therapeutic drugs were clearly determined to be lower than those of compounds having a sulfonyl fluoride group.

### [Example 7] (Not claimed)

A study was conducted on the optimum concentrations of PMSF, AEBSF and p-APMSF for inhibiting pancreatic juice protease.

More specifically, sample solutions to which were added various concentrations of PMSF (Roche), AEBSF (Roche) and p-APMSF (Sigma) were prepared for use as protease inhibitors, and the protease activity of each sample solution was measured in the same manner as Example 1 using pancreatin and two types of pancreatic juice specimens. The protease activity of a sample solution to which protease inhibitor was not added (Inhibitor (-)) was also measured in the same manner as a control.

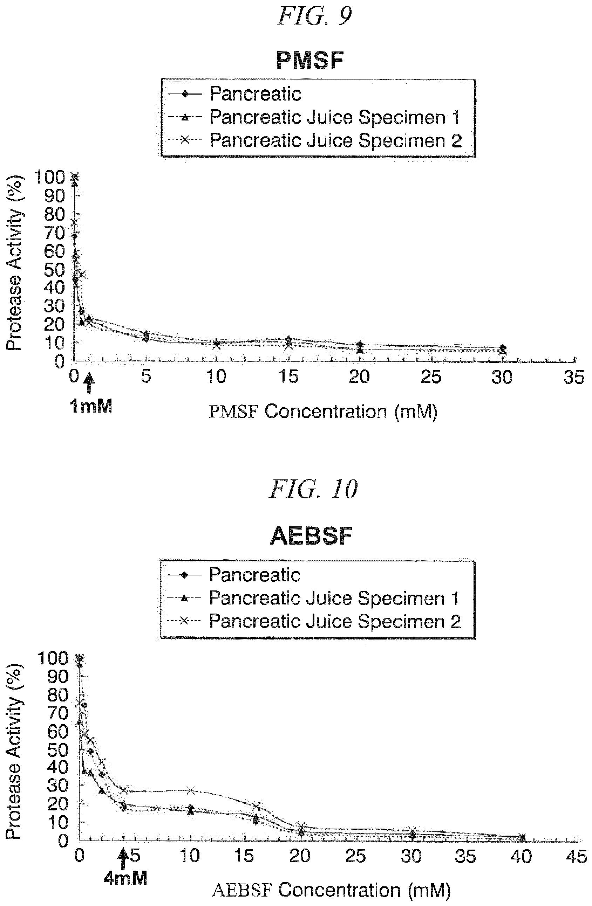

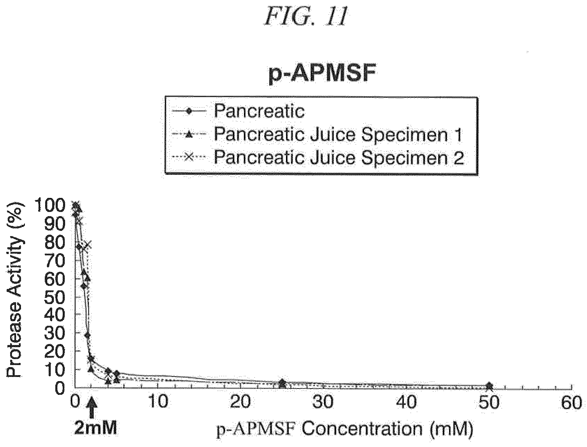

The results for the protease activity (relative value: %) of the sample solution to which PMSF was added are shown in FIG. 9, the results for the protease activity (relative value: %) of the sample solution to which AEBSF was added are shown in FIG. 10, and the results for the protease activity (relative value: %) of the sample solution to which p-APMSF was added are shown in FIG. 11. Protease activity based on a value of 100% for the fluorescence intensity of a sample solution to which protease inhibitor was not added is plotted on the vertical axis, and indicates relative values (%) of protease activity converted from the fluorescence intensity of each sample solution. The final concentrations of the protease inhibitor added to each sample solution are plotted on the horizontal axis. On the basis of these results, high levels of protease inhibitory effects against protease present in pancreatic juice were clearly demonstrated to be obtained in the case of a concentration of about 1 mM or higher for PMSF, a concentration of about 4 mM or higher for AEBSF, and a concentration of about 2 mM or higher for p-APMSF.

### [Example 8] (Not claimed)

A study was conducted of the effects on protease inhibitory activity observed as a result of combining PMSF, AEBSF and p-APMSF. PMSF (Roche), AEBSF (Roche) and p-APMSF (Sigma) were used as protease inhibitors.

More specifically, a sample solution obtained by adding 1 mM PMSF, a sample solution obtained by adding 4 mM AEBSF, a sample solution obtained by adding 2 mM p-APMSF, a sample solution obtained by adding 1 mM PMSF and 4 mM AEBSF, a sample solution obtained by adding 4 mM AEBSF and 2 mM p-APMSF, and a sample solution obtained by adding 1 mM PMSF and 2 mM p-APMSF were prepared as protease inhibitors, and the protease activity of each sample solution was measured in the same manner as Example 1 using three types of pancreatic juice specimens and two types of duodenal juice specimens. The protease activity of a sample solution to which protease inhibitor was not added (Inhibitor (-)) was also measured in the same manner as a control.

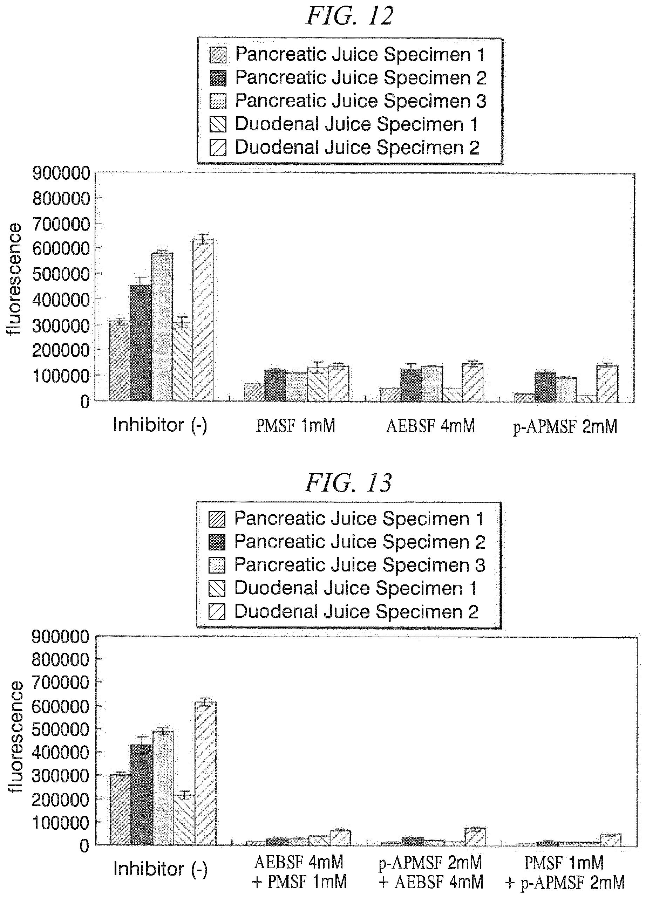

The results of measuring the fluorescence of the sample solutions to which were added one type of protease inhibitor are shown in FIG. 12, and the results of measuring the fluorescence of the sample solutions to which were added two types of protease inhibitors are shown in FIG. 13. Fluorescence plotted on the vertical axis represents the degraded amount of casein, and that value was estimated to be the value of protease activity. The protease inhibitors added to each sample solution are plotted on the horizontal axis. As a result, the case of combining addition of two or more types of protease inhibitors was clearly demonstrated to enhance protease inhibitory effects to a greater degree than in the case of adding one type of protease inhibitor. In addition, regardless of which combination of two types of PMSF, AEBSF and p-APMSF was used, roughly the same high levels of protease inhibitory effects were obtained.

### [Example 9]

According to the results of the aforementioned Example 8, protease inhibitory effects were clearly demonstrated to be enhanced by the combined addition of two or more types of protease inhibitors selected from the group consisting of PMSF, AEBSF and p-APMSF. Therefore, a study was conducted on whether or not protease inhibitory effects are enhanced by combining two types of these inhibitors with other inhibitors. PMSF (Roche), AEBSF (Roche), Aprotinin (Roche) and TLCK (Sigma) were used as protease inhibitors.

More specifically, a sample solution obtained by adding 1 mM PMSF and 4 mM AEBSF, a sample solution obtained by adding 1 mM PMSF, 4 mM AEBSF and 0.3 mM Aprotinin, and a sample solution obtained by adding 1 mM PMSF, 4 mM AEBSF and 1 mM TLCK were prepared as protease inhibitors, and the protease activity of each sample solution was measured in the same manner as Example 1 using four types of duodenal juice specimens. The protease activity of a sample solution to which protease inhibitor was not added (Inhibitor (-)) was also measured in the same manner as a control.

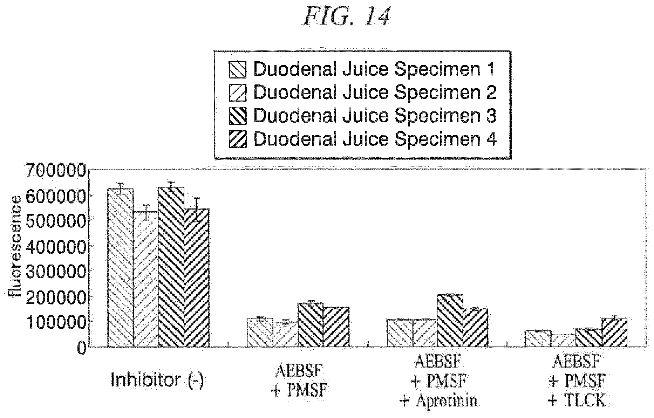

The results of measuring the fluorescence of sample solutions obtained by adding one type of other protease inhibitor not having a sulfonyl fluoride group to PMSF and AEBSF are shown in FIG. 14. Fluorescence plotted on the vertical axis represents the degraded amount of casein, and that value was estimated to be the value of protease activity. The protease inhibitors added to each sample solution are plotted on the horizontal axis. As a result, in the case of having added a peptide-based inhibitor in the form of Aprotinin to two or more types of protease inhibitors having a sulfonyl fluoride group, additional inhibitory effects were not observed in comparison with the case of adding PMSF and AEBSF only. In contrast, in the case of having added a sulfone-based protease inhibitor having a sulfonyl group in the form of TLCK to PMSF and AEBSF, additional inhibitory effects were observed in comparison with the case of adding only PMSF and AEBSF.

### INDUSTRIAL APPLICABILITY

Since the protease inhibition method of the present invention that uses the protease inhibitors of the present invention as defined in the claims is able to effectively inhibit protease activity in a biological sample such as pancreatic juice or duodenal juice, and enables biological components present in the biological sample to be preserved with greater stability, it can be used in fields relating to the analysis of biological samples such as pancreatic juice, and particularly in the field of clinical laboratory testing and the like.

## Claims

1. A method for inhibiting protease in a biological sample containing pancreatic juice components, comprising:
adding protease inhibitors comprising phenylmethylsulfonyl fluoride (PMSF), 4-(2-aminoethyl)-benzenesulfonyl fluoride (AEBSF) and N-α-tosyl-L-lysine chloromethyl ketone (TLCK) to the biological sample containing pancreatic juice components, thereby inhibiting protease enzyme activity in the biological sample.

2. The method for inhibiting protease in a biological sample containing pancreatic juice components according to claim 1, wherein PMSF, AEBSF, and TLCK is added to the biological sample so that the final concentration of PMSF is 1 mM or higher, the final concentration of AEBSF is 4 mM or higher, and the final concentration of TLCK is 0.1 mM or higher.

3. The method for inhibiting protease in a biological sample containing pancreatic juice components according to claim 1 or 2, wherein the biological sample is pancreatic juice or duodenal juice.

4. A protease inhibitor mixture comprising phenylmethylsulfonyl fluoride (PMSF), 4-(2-aminoethyl)-benzenesulfonyl fluoride (AEBSF) and N-α-tosyl-L-lysine chloromethyl ketone (TLCK).

## Patentansprüche

1. Verfahren zur Hemmung von Protease in einer biologischen Probe, die Pankreassaftkomponenten enthält, umfassend:
Zugeben von Proteasehemmern, die Phenylmethylsulfonylfluorid (PMSF), 4-(2-Aminoethyl)benzolsulfonylfluorid (AEBSF) und N-α-Tosyl-L-lysinchlormethylketon (TLCK) umfassen, zu der biologischen Probe, die Pankreassaftkomponenten enthält, wodurch die Protease-Enzymaktivität in der biologischen Probe gehemmt wird.

2. Verfahren zur Hemmung von Protease in einer biologischen Probe, die Pankreassaftkomponenten enthält, nach Anspruch 1, wobei PMSF, AEBSF und TLCK der biologischen Probe zugegeben werden, so dass die Endkonzentration von PMSF 1 mM oder höher ist, die Endkonzentration von AEBSF 4 mM oder höher ist und die Endkonzentration von TLCK 0,1 mM oder höher ist.

3. Verfahren zur Hemmung von Protease in einer biologischen Probe, die Pankreassaftkomponenten enthält, nach Anspruch 1 oder 2, wobei die biologische Probe Pankreassaft oder Duodenalsaft ist.

4. Proteasehemmermischung, die Phenylmethylsulfonylfluorid (PMSF), 4-(2-Aminoethyl)benzolsulfonylfluorid (AEBSF) und N-α-Tosyl-L-lysinchlormethylketon (TLCK) umfasst.

## Revendications

1. Procédé pour inhiber une protéase dans un échantillon biologique contenant des composants de suc pancréatique, comprenant :
ajouter des inhibiteurs de protéase comprenant le fluorure de phénylméthylsulfonyle (PMSF), le fluorure de 4-(2-aminoéthyl)-benzènesulfonyle (AEBSF) et la N-α-tosyl-L-lysine chlorométhyl cétone (TLCK) à l'échantillon biologique contenant des composants de suc pancréatique, permettant ainsi d'inhiber l'activité de l'enzyme protéase dans l'échantillon biologique.

2. Procédé pour inhiber une protéase dans un échantillon biologique contenant des composants de suc pancréatique selon la revendication 1, dans lequel le PMSF, l'AEBSF et la TLCK sont ajoutés à l'échantillon biologique de telle sorte que la concentration finale de PMSF est 1 mM ou plus, la concentration finale d'AEBSF est 4 mM ou plus et la concentration finale de TLCK est 0,1 mM ou plus.

3. Procédé pour inhiber une protéase dans un échantillon biologique contenant des composants de suc pancréatique selon l'une des revendications 1 ou 2, dans lequel l'échantillon biologique est un suc pancréatique ou un suc duodénal.

4. Mélange d'inhibiteurs de protéase comprenant le fluorure de phénylméthylsulfonyle (PMSF), le fluorure de 4-(2-aminoéthyl)-benzènesulfonyle (AEBSF) et la N-α-tosyl-L-lysine chlorométhyl cétone (TLCK).
